# FASCICULE DE BREVET EUROPEEN

(11) **EP 3 746 148 B1**
(45) Date de publication et mention de la délivrance du brevet: **10.12.2025**
(21) Numéro de dépôt: 19707422.2
(22) Date de dépôt: 28.01.2019
(51) Int. Cl.: A61M 60/174, A61M 60/237, A61M 60/863, A61M 60/861

(54) **ENSEMBLE D'ASSISTANCE VENTRICULAIRE À POMPE CARDIAQUE STABILISÉE**
VENTRIKULÄRE UNTERSTÜTZUNGSVORRICHTUNG MIT STABILISIERTER HERZPUMPE
VENTRICULAR ASSISTANCE ASSEMBLY WITH STABILISED CARDIAC PUMP

(30) Priorité: 29.01.2018 FR 1850699
(43) Date de publication de la demande: 09.12.2020
(73) Titulaire: Fineheart, 33600 Pessac (FR)
(72) Inventeur: GARRIGUE, Stéphane, 33130 BEGLES (FR); MASCARELL, Arnaud, 37250 MONTBAZON (FR)
(74) Mandataire: Plasseraud IP
(86) Numéro de dépôt international: PCT/FR2019/050176
(87) Numéro de publication internationale: WO 2019/145657

(56) Documents cités:
- WO-A1-2010/010407
- WO-A2-2016/005803
- US-A1- 2010 249 489

## Description

### ARRIERE-PLAN DE L'INVENTION

### Domaine de l'invention

La présente invention concerne un ensemble d'assistance ventriculaire comprenant un dispositif de stabilisation d'une pompe cardiaque afin de maintenir la partie de cette dernière, placée dans un ventricule d'un cœur battant, centrée sur la valve correspondante.

### Arrière-plan technologique

L'insuffisance cardiaque (IC), est un état pathologique dans lequel le cœur d'un patient présente une incapacité à fournir un débit sanguin nécessaire aux besoins métaboliques de l'organisme.

Il est connu pour traiter l'insuffisance cardiaque d'implanter un dispositif d'assistance ventriculaire (DAV), qui est une pompe cardiaque artificielle.

Cette pompe mécanique ne remplace pas le cœur qui continue à fonctionner, mais apporte une aide au ventricule affaibli afin d'accroître le débit sanguin de façon adaptée aux besoins de l'individu.

Cette assistance peut être temporaire dans l'attente d'un greffon disponible pour réaliser une transplantation cardiaque.

Cependant, on observe une proportion significative de patients qui ne recevront pas un tel greffon, soit parce qu'ils ne peuvent être candidats à une telle transplantation, par exemple en raison d'une insuffisance cardiaque sévère, soit parce qu'aucun greffon adapté n'est disponible pour ces patients.

Dans ce cas, l'assistance ventriculaire est utilisée en destination, c'est-à-dire que la pompe cardiaque artificielle est implantée à long terme.

Ces pompes cardiaques font donc l'objet d'intenses recherches visant à améliorer la survie et la qualité de vie des patients présentant une insuffisance cardiaque.

De nombreuses avancées ont été réalisées ces dernières années et on connaît aujourd'hui des dispositifs d'assistance ventriculaire plus compacts, silencieux et présentant une durée de service accrue.

Les pompes cardiaques implantables de l'état de l'art sont ainsi typiquement équipées d'un moteur électrique intégré pour assurer leur fonctionnement, la vitesse de rotation de la pompe fournissant la force nécessaire pour faire circuler le sang depuis le ventricule affaibli vers la circulation corporelle.

Le contrôleur et la source d'alimentation de la pompe cardiaque sont typiquement placés à l'extérieur du patient. Une ligne percutanée au niveau de l'abdomen assure alors la liaison entre la pompe fixée à la paroi du ventricule et ces éléments externes.

Le document WO2016005803 A2 décrit un ensemble d'assistance ventriculaire pour assister un cœur selon le préambule de la revendication indépendante 1.

La demande de brevet WO2013014339 A1 au nom de la présente demanderesse décrit une pompe cardiaque électrique particulièrement fiable et aisée d'installation dont l'unité de contrôle et sa batterie électrique sont implantées.

Bien que représentant un progrès certain pour la qualité de vie d'un patient souffrant d'insuffisance cardiaque, le fonctionnement de ces pompes cardiaques peut encore être amélioré.

En effet, de telles pompes cardiaques présentent typiquement un carter, ou corps de pompe, dont l'extrémité distale est formée par un conduit de petit diamètre au travers duquel le sang est éjecté vers la valve cardiaque correspondante, généralement la valve aortique.

Or, en raison, non seulement des gestes du quotidien réalisés par un patient équipé d'un tel dispositif d'assistance ventriculaire, mais également des chocs que ce dernier peut subir dans ses activités, on observe que l'extrémité distale de la pompe cardiaque est susceptible de bouger jusqu'à ne plus être placée en regard de la valve correspondante, notamment la valve aortique.

Non seulement le débit de sang passant au travers de cette valve est alors réduit, mais l'extrémité distale de la pompe peut, de manière extrême, entrer en contact avec une paroi du cœur et provoquer la formation d'un thrombus, ou caillot, particulièrement dangereux pour le patient.

Il existe donc un besoin pressant pour un dispositif permettant de stabiliser en position la partie d'une pompe cardiaque placée dans un ventricule d'un cœur battant afin de maintenir son centrage sur la valve correspondante.

### Objet de l'invention

La présente invention vise à pallier les inconvénients de l'art antérieur et à répondre aux contraintes ci-dessus énoncées en proposant un dispositif pour maintenir l'extrémité libre, ou conduit d'éjection du sang, d'une pompe cardiaque implantable et destinée à une mise en place intraventriculaire, qui soit simple dans sa conception et dans son mode opératoire, biocompatible et particulièrement fiable mécaniquement.

La présente invention vise notamment un tel dispositif de stabilisation de l'extrémité libre d'une pompe cardiaque, permettant de maintenir fermement en position centrée, cette extrémité libre sur la valve correspondante du cœur humain.

Un autre objet de la présente invention est un tel dispositif de stabilisation qui autorise au praticien d'intervenir sur la pompe cardiaque depuis l'extérieur du ventricule, par exemple pour un remplacement de la pompe ou d'un de ses composants.

La présente invention vise également un procédé de stabilisation d'une pompe cardiaque destinée à assister un cœur humain qui soit simple et assure une intervention sur la pompe cardiaque sans nécessité d'intervention chirurgicale lourde.

### BREVE DESCRIPTION DE L'INVENTION

A cet effet, l'invention concerne un ensemble d'assistance ventriculaire pour assister un cœur, ledit ensemble comprenant :
- un dispositif d'ancrage d'une pompe cardiaque, destiné à être assemblé à une ouverture d'une paroi ventriculaire dudit cœur, ledit dispositif d'ancrage délimitant un passage interne,
- une pompe cardiaque destinée à être fixée sur ledit dispositif d'ancrage, ladite pompe cardiaque étant configurée pour une mise en place intraventriculaire dans ledit cœur, ladite pompe cardiaque ayant une extrémité distale,
- ladite pompe s'étendant au travers du passage interne du dispositif d'ancrage à l'intérieur de ce cœur lorsqu'elle est fixée audit dispositif d'ancrage implanté dans ladite ouverture, de sorte que son extrémité distale est placée dans une chambre ventriculaire,
- un dispositif de stabilisation de cette pompe cardiaque comprenant au moins deux organes de liaison allongés, biocompatibles et flexibles, chaque organe de liaison allongé étant destiné à relier une partie de ladite pompe cardiaque placée dans ladite chambre ventriculaire à une paroi interne de ladite chambre ventriculaire.

Cette pompe cardiaque étant ancrée dans la paroi du cœur avec une mise en place intraventriculaire, et son extrémité, ou conduit d'éjection du sang, étant stabilisée par le dispositif de stabilisation dans une position optimale par rapport à la valve correspondante, le patient peut dès lors se déplacer de manière active sans aucun risque.

L'extrémité libre de la pompe cardiaque reste ainsi centrée sur la valve correspondante, notamment la valve aortique.

Dans le contexte de la présente invention, le terme « proximal » signifie la position la plus proche du professionnel de santé, ou du praticien, tandis que le terme « distal » doit ici être entendu comme signifiant le plus éloigné de ce professionnel. En d'autres termes, l'extrémité distale d'une tige, ou d'un câble allongé, est l'extrémité qui est la première engagée dans un canal de passage de câble de la pompe cardiaque tandis que l'extrémité proximale serait la dernière extrémité engagée.

Dans différents modes de réalisation particuliers de cet ensemble, chacun ayant ses avantages particuliers et susceptibles de nombreuses combinaisons techniques possibles:
- pour chaque organe de liaison allongé, ledit ensemble comprend un élément de fixation afin d'assurer l'ancrage dudit organe de liaison dans ladite paroi interne.

De préférence, cet élément de fixation est une vis d'ancrage prolongeant axialement ledit organe de liaison allongé et placée à l'extrémité distale de ce dernier.
- chaque organe de liaison allongé est choisi dans le groupe comprenant un câble allongé, un fil, une cordelette, une tige et des combinaisons de ces éléments,
- ledit ensemble comprend également un anneau, ou une collerette, destiné à entourer l'extrémité distale de ladite pompe cardiaque, au moins certains desdits organes de liaison allongés étant solidaires par une de leurs extrémités de cet anneau, ou respectivement de cette collerette.

De manière avantageuse, ces organes de liaison allongés étant solidaires de l'anneau ou de la collerette, l'ensemble formé dudit anneau ou de ladite collerette et desdits organes de liaison, est réalisé dans un matériau à mémoire de forme de sorte qu'étant introduit dans la chambre, cet ensemble passe d'une configuration non déployée à une configuration déployée dans laquelle les extrémités libres des organes de liaison viennent en appui contre les parois internes de ladite chambre ventriculaire.

Le matériau utilisé peut être un alliage à mémoire de forme (SMAs - "shape-memory alloys"), le plus couramment utilisé étant le nitinol. Un tel matériau présente l'avantage d'être résistant, léger tout en présentant cette particularité d'être apte à se déformer à une température donnée, et de recouvrir sa forme initiale, une forme non déformée, lorsque sa température atteint une température supérieure à une température dite de transformation.
- chaque organe de liaison allongé et flexible étant un câble allongé ou une tige, ladite pompe cardiaque comporte des canaux de passage de câble configurés pour permettre chacun l'insertion d'un câble allongé, ou d'une tige, correspondant depuis l'extérieur du cœur vers l'intérieur de ladite chambre ventriculaire lorsque ladite pompe est fixée e au dispositif d'ancrage.

De préférence, le corps de chaque câble allongé, ou tige, est configuré pour présenter en torsion une rigidité suffisante pour assurer la transmission sur toute sa longueur d'un mouvement de rotation imprimé depuis une extrémité proximale de ce câble ou de cette tige.

De manière avantageuse, les canaux de passage de câble s'étendent depuis l'extrémité proximale de ladite pompe cardiaque jusqu'à une partie de ladite pompe destinée à être placée à l'intérieur de ladite chambre, lorsque ladite pompe est fixée au dispositif d'ancrage solidaire de ladite ouverture. A titre purement illustratif, ces canaux de passage peuvent déboucher au niveau de l'extrémité distale de ladite pompe cardiaque.

Ainsi, et de manière extrêmement avantageuse, le dispositif de stabilisation est entièrement accessible depuis l'extérieur du ventricule, et le praticien peut manipuler les câbles allongés ou tiges depuis l'extérieur du cœur, la pompe cardiaque étant en position assemblée à son anneau d'ancrage.

La pompe cardiaque est donc aisément interchangeable et d'une maintenance simplifiée sans requérir de geste chirurgical lourd.

Le remplacement de la pompe cardiaque reste de ce fait particulièrement aisé en cas de panne ou d'usure d'un composant.

De manière préférentielle, au moins une partie d'au moins un desdits canaux de passage est placée dans l'épaisseur de la paroi latérale délimitant le corps de pompe et/ou est délimitée par un élément creux et allongé formant saillie du corps de pompe, c'est-à-dire placé à l'extérieur du corps de pompe.

Dans ce dernier cas, et à titre d'exemple, il peut s'agir d'un élément ayant une section transversale droite en forme de U ou demi-rond.

Bien entendu, le dispositif d'ancrage peut être configuré pour permettre l'insertion et le passage d'une pompe cardiaque dont le corps de pompe présente un ou plusieurs éléments formant saillie tout en assurant l'étanchéité nécessaire. Par exemple, la paroi intérieure du dispositif d'ancrage délimitant l'orifice pour le passage du corps de pompe, peut présenter des creux dont les formes sont complémentaires de celles des éléments formant saillie du corps de pompe.

Selon un mode de réalisation particulièrement avantageux, chaque canal de passage comporte une valve anti-retour pour assurer son étanchéité.
- ladite pompe est une pompe cardiaque propulsive.

La présente invention concerne également un système d'assistance ventriculaire d'un cœur comprenant un ensemble d'assistance ventriculaire tel que décrit précédemment.

Selon l'invention,
- le dispositif d'ancrage de ladite pompe cardiaque étant assemblé à une ouverture réalisée dans la paroi ventriculaire dudit cœur,
- ladite pompe étant reliée audit dispositif d'ancrage de sorte qu'une partie de ladite pompe passant au travers d'un orifice délimité par ledit dispositif d'ancrage, a son extrémité proximale placée extérieurement audit cœur et son extrémité distale placée dans une chambre ventriculaire dudit coeur,
- ladite pompe présentant des canaux de passage de câble allongé configurés pour permettre chacun l'insertion d'un câble allongé et flexible ou d'une tige depuis l'extérieur du cœur vers l'intérieur de ladite chambre,
- un premier câble allongé, ou une première tige, passant par un premier desdits canaux placé sur un premier bord de ladite pompe, a son extrémité distale ancrée dans le tissu d'une paroi de septum de ce cœur et un deuxième câble allongé, ou une deuxième tige, passant par un deuxième desdits canaux placé sur le bord de ladite pompe opposé au bord recevant ledit premier canal, a son extrémité distale ancrée dans le tissu d'une paroi interne à ladite chambre ventriculaire et contigüe à la valve aortique de sorte que l'extrémité distale de ladite pompe cardiaque est stabilisée en position dans ladite chambre ventriculaire.

Bien entendu, on cherchera à ancrer lesdits câbles allongés et flexibles, ou tiges, dans la chambre ventriculaire de sorte que l'extrémité distale de ladite pompe soit placée en regard de la valve aortique et centrée sur celle-ci.

De même, la partie de la pompe cardiaque placée dans la chambre peut être reliée par plus de deux câbles allongés et/ou tiges.

La présente invention concerne encore un procédé de stabilisation d'une pompe cardiaque destinée à assister un cœur, ladite pompe ayant une extrémité distale et une extrémité proximale, dans lequel après avoir assemblé un dispositif d'ancrage de ladite pompe cardiaque à une ouverture réalisée dans la paroi ventriculaire dudit cœur, et relié ladite pompe audit dispositif d'ancrage de sorte qu'une partie de ladite pompe passant au travers d'un orifice délimité par ledit dispositif d'ancrage, a son extrémité proximale placée extérieurement audit cœur et son extrémité distale placée dans une chambre ventriculaire.

Selon l'invention,
- ladite pompe présentant des canaux de passage de câble allongé et flexible configurés pour permettre l'insertion de tels câbles, ou de tiges, depuis l'extérieur du cœur vers l'intérieur de ladite chambre lorsque ladite pompe est fixée au dispositif d'ancrage,
- on réalise les étapes suivantes :
   * introduire depuis l'extérieur du cœur un premier câble allongé, ou une première tige, dans un premier canal de passage jusqu'à ce que l'extrémité libre dudit premier câble, ou de la première tige, ressorte dans la chambre ventriculaire, puis approcher ladite extrémité libre d'une paroi de septum de ce cœur et fixer celle-ci dans ladite paroi de septum,
   * introduire depuis l'extérieur du cœur, un deuxième câble allongé, ou une seconde tige, dans un second canal de passage jusqu'à ce que l'extrémité libre dudit deuxième câble, ou de la seconde tige, ressorte dans la chambre ventriculaire, puis approcher ladite extrémité libre d'une paroi interne à ladite chambre ventriculaire et contigüe à la valve aortique et fixer celle-ci dans ladite paroi, puis éventuellement
- mettre sous tension lesdits câbles allongés pour stabiliser l'extrémité libre de ladite pompe cardiaque dans ladite chambre.

A titre d'exemple, cette mise sous tension peut être réalisée en exerçant une traction sur les câbles allongés, ou sur les tiges, du côté de l'extrémité proximale de ladite pompe cardiaque.

Bien entendu, on cherchera à disposer lesdits câbles allongés, ou tiges, dans la chambre ventriculaire de sorte que l'extrémité distale de ladite pompe soit placée en regard de la valve aortique et centrée sur celle-ci.

Selon un mode de réalisation de ce procédé, on introduit au moins un troisième câble allongé, ou une troisième tige, dans un canal de passage correspondant de la pompe cardiaque jusqu'à ce que l'extrémité libre dudit troisième câble, ou de la troisième tige, ressorte dans la chambre ventriculaire, puis on approche ladite extrémité libre d'une paroi interne à ladite chambre ventriculaire et on fixe celle-ci dans ladite paroi.

Cette introduction des câbles, ou des tiges, peut être réalisée de manière axiale par rapport au corps de pompe ou ceux-ci peuvent être introduits latéralement.

De préférence, chaque canal de passage est configuré pour coopérer avec son câble allongé pour assurer l'étanchéité, une fois le câble en position. Ainsi, et selon un autre mode de réalisation de ce procédé, chaque canal de passage comporte une valve anti-retour pour assurer son étanchéité avant introduction d'un câble allongé ou d'une tige.

De manière avantageuse, chaque câble allongé comportant une pointe de perçage à son extrémité libre, on perce le tissu de chaque paroi pour assurer l'ancrage du câble allongé correspondant.

Avec un tel dispositif de stabilisation, le praticien peut avantageusement orienter l'extrémité libre du câble allongé et flexible, ou de la tige, en direction d'une paroi de la chambre ventriculaire, jusqu'à accoster cette paroi, où il pourra ensuite ancrer l'extrémité libre de celui-ci par vissage de cette extrémité libre dans le tissu de la paroi.

Selon encore un autre mode de réalisation de ce procédé, lesdits canaux de passage s'étendent depuis l'extrémité proximale de ladite pompe, à l'extérieur du cœur, jusqu'à une partie de ladite pompe destinée à être placée à l'intérieur de ladite chambre lorsque ladite pompe est fixée au dispositif d'ancrage.

Ainsi, la manipulation des câbles allongés et/ou des tiges peut être réalisée par le praticien, alors que la pompe cardiaque est fixée sur son dispositif d'ancrage. Il en résulte une grande facilité de maintenance de la pompe cardiaque qui peut aisément être manipulée par le praticien sans nécessité d'intervention chirurgicale lourde pour le patient afin d'accéder au dispositif de stabilisation.

A titre purement illustratif, ces canaux de passage peuvent déboucher au niveau de l'extrémité distale de ladite pompe cardiaque.

Selon encore un autre mode de réalisation de ce procédé, chaque canal de passage est placé au moins en partie dans l'épaisseur de la paroi latérale délimitant le corps de pompe et/ou est délimité par un élément formant saillie du corps de pompe, ledit élément étant creux et allongé.

Dans ce dernier cas, il peut s'agir par exemple d'un élément ayant une section transversale droite en forme de U ou demi-rond.

Le dispositif d'ancrage peut être configuré pour permettre l'insertion et le passage d'une pompe cardiaque, dont le corps de pompe présente un ou plusieurs éléments formant saillie tout en assurant l'étanchéité nécessaire. Par exemple la paroi intérieure délimitant l'orifice pour le passage du corps de pompe peut présenter des creux dont les formes sont complémentaires de celles des éléments formant saillie du corps de pompe.

### BREVE DESCRIPTION DES DESSINS

D'autres avantages, buts et caractéristiques particulières de la présente invention ressortiront de la description qui va suivre, faite, dans un but explicatif et nullement limitatif, en regard des dessins annexés, dans lesquels:
- la Figure 1 représente schématiquement un ensemble d'assistance ventriculaire d'un cœur humain selon un mode de réalisation particulier de l'invention, la pompe cardiaque étant insérée dans le ventricule gauche de ce cœur ;
- la Figure 2 est une vue schématique et en coupe transversale droite du seul carter, ou corps de pompe, de la pompe cardiaque de la Fig. 1 montrant les canaux de passage de câble ;
- la Figure 3 montre un câble allongé mise en œuvre dans l'ensemble d'assistance ventriculaire de la Fig. 1 et destiné à être inséré dans un des canaux de passage de câble du corps de pompe ;
- la Figure 4 est une vue schématique et en coupe transversale droite du seul carter, ou corps de pompe, d'une pompe cardiaque d'un ensemble d'assistance ventriculaire selon un autre mode de réalisation de l'invention.

### DESCRIPTION DETAILLEE DE MODE DE REALISATION DE L'INVENTION

Tout d'abord, on note que les figures ne sont pas à l'échelle.

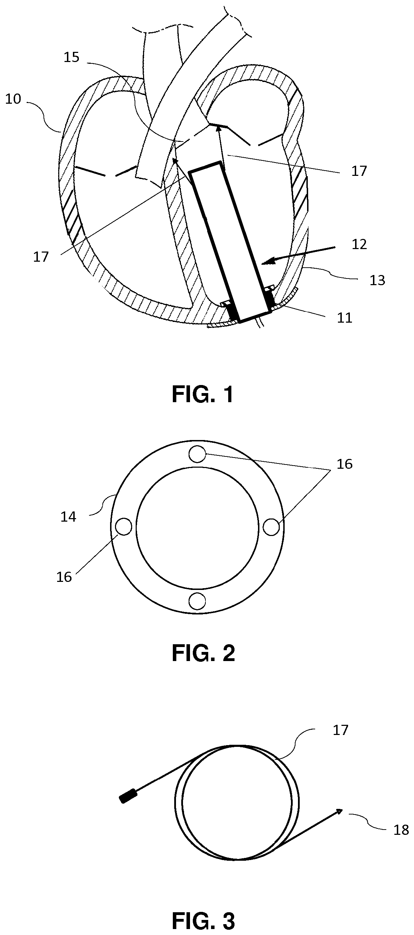

Les Figures 1 à 3 montrent de manière schématique un ensemble d'assistance ventriculaire d'un cœur 10 humain selon un mode de réalisation particulier de la présente invention.

Cet ensemble comprend une bague 11 d'ancrage d'une pompe cardiaque 12, assemblée à une ouverture réalisée dans la paroi ventriculaire 13 de ce cœur 10.

Cette bague 11 d'ancrage comporte à ses extrémités des collerettes, ou brides, venant prendre appui de part et d'autre de la paroi ventriculaire 13 pour fixer à demeure cette bague 11 à la paroi ventriculaire 13.

La collerette destinée à être plaquée contre la surface intérieure de la paroi ventriculaire 13 est de préférence réalisée dans un matériau à mémoire de forme tel qu'en nitinol, de manière à pouvoir traverser l'ouverture réalisée dans la paroi ventriculaire 13 dans un état déformé, dans lequel elle présente une forme tubulaire, ou sensiblement tubulaire, et à reprendre sa forme initiale, celle d'une bride, après introduction dans la chambre ventriculaire correspondante.

La bague 11 d'ancrage comporte un orifice pour assurer le passage d'une partie de la pompe cardiaque 12 lors de sa fixation sur cette bague 11 d'ancrage.

Cette pompe cardiaque 12 est de plus configurée pour une mise en place intraventriculaire, c'est-à-dire que l'extrémité distale du corps 14 de pompe, ou carter, est destinée à être positionnée dans la chambre ventriculaire correspondante une fois cette pompe fixée sur la bague 11 d'ancrage.

Ainsi, cette pompe cardiaque 12 étant reliée à la bague 11 d'ancrage, une partie du corps 14 de pompe passe au travers de l'orifice délimité par la bague 11 d'ancrage, tandis que l'extrémité proximale du corps 14 de pompe est placée extérieurement, ou est affleurant, à la paroi ventriculaire 13. L'extrémité distale est placée dans la chambre ventriculaire en amont de la valve 15 aortique, par rapport au sens d'éjection du sang.

Le corps 14 de pompe définit également un logement pour recevoir un moteur (non représenté) qui est disposé dans la chambre ventriculaire et/ou dans l'épaisseur de la paroi ventriculaire 13, de façon à aspirer puis refouler le sang, depuis le fond, dans la chambre ventriculaire et dans la direction de la valve aortique 15, à travers le corps 14 de pompe.

Afin de stabiliser le corps 14 de pompe dans la chambre ventriculaire, dans une position optimale en regard de la valve aortique 15, le corps 14 de pompe, ou carter, présente des canaux 16 de passage de câble allongé, lesquels sont configurés pour permettre chacun l'insertion d'un câble 17 allongé et flexible depuis l'extérieur du cœur vers l'intérieur de la chambre ventriculaire.

Ainsi, ces canaux 16 de passage s'étendant axialement depuis l'extrémité proximale du corps de pompe jusqu'à son extrémité distale, les câbles 17 allongés et flexibles peuvent être manipulés depuis l'extérieur du ventricule.

La Figure 2 montre une vue en coupe transversale droite du seul corps 14 de pompe, lequel présente dans son épaisseur des canaux 16 de passage régulièrement répartis sur le pourtour de ce corps.

Chaque canal 16 de passage comporte au moins un élément d'étanchéité (non représenté) tel qu'une valve anti-retour, pour assurer l'étanchéité du canal avant insertion d'un câble 17 allongé et flexible.

Comme représenté à la Figure 1, un premier câble 17 allongé et flexible, passe par un premier de ces canaux 16 de passage de câble placé sur un premier bord de ladite pompe cardiaque 12 et ressort de ce corps par l'extrémité distale du corps 14 de pompe.

L'extrémité libre de ce câble 17, qui comporte une vis hélicoïdale 18 saillante placée dans le prolongement axial du câble et apte à pénétrer dans le tissu d'une paroi du cœur sous l'effet d'un mouvement de vissage imprimé depuis l'extrémité proximale du câble allongé, est ancrée dans le tissu d'une paroi septale du ventricule gauche de ce cœur ("Left septal wall").

Un deuxième câble 17 allongé et flexible passant par un deuxième canal 16 placé sur le bord de cette pompe 12 opposé au bord recevant le premier canal, a son extrémité distale ancrée dans le tissu d'une paroi interne à ladite chambre ventriculaire et attenante à la valve aortique 15. A titre d'exemple, cette dernière paroi est la chambre de chasse de la valve aortique ("Aortic outflow track").

Ces câbles 17 étant sous tension, la pompe cardiaque 12 est bloquée en position sur le long terme et ce, quelle que soit l'activité réalisée par le patient équipé de cet ensemble d'assistance ventriculaire.

Alors que le débit sanguin au travers de la valve aortique 15 est rendu optimal car l'extrémité distale du corps 14 de pompe est centrée sur la valve aortique 15, on évite également toute formation éventuelle d'un caillot.

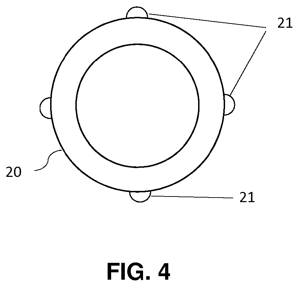

La Figure 4 est une vue schématique et en coupe du seul carter, ou corps de pompe, d'une pompe cardiaque d'un ensemble d'assistance ventriculaire selon un autre mode de réalisation de l'invention.

Ce corps de pompe 20 comporte une pluralité de canaux 21 de passage de câble allongé, lesquels sont placés extérieurement à la pompe.

A titre purement illustratif, ces canaux 21 de passage sont par exemple définis par des protubérances allongées et creuses formées sur la surface extérieure du corps de pompe.

Bien entendu, la paroi interne délimitant l'orifice de la bague d'ancrage correspondante comprend des creux dont la forme est complémentaire de ces protubérances, de manière à assurer le passage de la pompe cardiaque au travers de l'orifice lors de sa fixation sur cette bague d'ancrage.

Ces creux sont également configurés pour assurer par coopération avec ces protubérances l'étanchéité requise à l'assemblage de la pompe cardiaque et de la bague d'ancrage.

## Revendications

1. Ensemble d'assistance ventriculaire pour assister un cœur, ledit ensemble comprenant :
- un dispositif d'ancrage (11) d'une pompe cardiaque (12), destiné à être assemblé à une ouverture d'une paroi ventriculaire (13) dudit cœur, ledit dispositif d'ancrage (11) délimitant un passage interne,
- une pompe cardiaque (12) destinée à être fixée sur ledit dispositif d'ancrage (11), ladite pompe cardiaque (12) étant configurée pour une mise en place intraventriculaire dans ledit cœur, ladite pompe cardiaque (12) ayant une extrémité distale,
- ladite pompe cardiaque (12) s'étendant au travers dudit passage interne à l'intérieur dudit cœur lorsqu'elle est fixée audit dispositif d'ancrage (11) implanté dans ladite ouverture de sorte que son extrémité distale est placée dans une chambre ventriculaire, **caractérisé en ce que** ledit ensemble comprend:
- un dispositif de stabilisation de ladite pompe cardiaque (12) comprenant au moins deux organes de liaison allongés, biocompatibles et flexibles, chaque organe de liaison (17) allongé étant destiné à relier une partie de ladite pompe cardiaque (12) placée dans ladite chambre ventriculaire à une paroi interne de ladite chambre ventriculaire.

2. Ensemble selon la revendication 1, **caractérisé en ce que** pour chaque organe de liaison (17) allongé, ledit ensemble comprend un élément de fixation afin d'assurer l'ancrage dudit organe de liaison (17) dans ladite paroi interne.

3. Ensemble selon la revendication 2, **caractérisé en ce que** ledit élément de fixation est une vis d'ancrage prolongeant axialement ledit organe de liaison (17) allongé et placée à l'extrémité distale de ce dernier.

4. Ensemble selon l'une quelconque des revendications 1 à 3, **caractérisé en ce que** chaque organe de liaison (17) allongé est choisi dans le groupe comprenant un câble allongé, un fil, une cordelette, une tige et des combinaisons de ces éléments.

5. Ensemble selon l'une quelconque des revendications 1 à 4, **caractérisé en ce que** ledit ensemble comprend également un anneau, ou une collerette, destiné à entourer l'extrémité distale de ladite pompe cardiaque (12), au moins certains desdits organes de liaison allongés étant solidaires par une de leurs extrémités de cet anneau, ou respectivement de cette collerette.

6. Ensemble selon la revendication 5, **caractérisé en ce que** lesdits organes de liaison allongés étant solidaires dudit anneau ou de ladite collerette, l'ensemble formé dudit anneau ou de ladite collerette et desdits organes de liaison, est réalisé dans un matériau à mémoire de forme de sorte qu'étant introduit dans la chambre, cet ensemble passe d'une configuration non déployée à une configuration déployée dans laquelle les extrémités libres des organes de liaison viennent en appui contre les parois internes de ladite chambre ventriculaire.

7. Ensemble selon l'une quelconque des revendications 1 à 5, **caractérisé en ce que** chaque organe de liaison (17) allongé étant un câble allongé ou une tige, ladite pompe cardiaque (12) comporte des canaux de passage (16) de câble configurés pour permettre chacun l'insertion d'un câble allongé, ou d'une tige, correspondant depuis l'extérieur du cœur vers l'intérieur de ladite chambre ventriculaire lorsque ladite pompe est fixée e au dispositif d'ancrage (11).

8. Ensemble selon la revendication 7, **caractérisé en ce que** le corps de chaque câble allongé ou tige est configuré pour présenter en torsion une rigidité assurant la transmission sur toute sa longueur d'un mouvement de rotation imprimé depuis une extrémité proximale de ce câble ou de cette tige.

9. Ensemble selon la revendication 7 ou 8, **caractérisé en ce que** les canaux de passage (16) de câble s'étendent depuis l'extrémité proximale de ladite pompe cardiaque (12) jusqu'à une partie de ladite pompe destinée à être placée à l'intérieur de ladite chambre lorsque ladite pompe est fixée au dispositif d'ancrage (11) solidaire de ladite ouverture.

10. Ensemble selon l'une quelconque des revendications 7 à 9, **caractérisé en ce que** au moins une partie d'au moins un desdits canaux de passage (16) est placée dans l'épaisseur de la paroi latérale délimitant le corps de pompe et/ou est délimitée par un élément creux et allongé formant saillie du corps de pompe.

11. Ensemble selon l'une quelconque des revendications 7 à 10, **caractérisé en ce que** chaque canal de passage comporte une valve anti-retour pour assurer son étanchéité.

12. Ensemble selon l'une quelconque des revendications 1 à 11, **caractérisé en ce que** ladite pompe est une pompe cardiaque (12) propulsive.

## Patentansprüche

1. Ventrikuläre Herzunterstützungsanordnung zur Unterstützung eines Herzens, wobei die Vorrichtung umfasst:
- eine Verankerungsvorrichtung (11) für eine Herzpumpe (12), die dazu bestimmt ist, an einer Öffnung einer Ventrikelwand (13) des Herzens angebracht zu werden, wobei die Verankerungsvorrichtung (11) einen Durchgang begrenzt,
- eine Herzpumpe (12), die dazu bestimmt ist, an der Verankerungsvorrichtung (11) befestigt zu werden, wobei die Herzpumpe (12) für eine intraventrikuläre Platzierung in dem Herzen ausgebildet ist und ein distales Ende aufweist,
- wobei sich die Herzpumpe (12) durch den Durchgang innerhalb des Herzens erstreckt, wenn sie an der in der Öffnung implantierten Verankerungsvorrichtung (11) befestigt ist, so dass ihr distales Ende in einer ventrikulären Kammer angeordnet ist,
**dadurch gekennzeichnet, dass** die Anordnung umfasst:
- eine Vorrichtung zur Stabilisierung der Herzpumpe (12), die wenigstens zwei längliche, biokompatible und flexible Verbindungselemente umfasst, wobei jedes längliche Verbindungselement (17) dazu bestimmt ist, einen Teil der in der ventrikulären Kammer platzierten Herzpumpe (12) mit einer inneren Wand der ventrikulären Kammer zu verbinden.

2. Anordnung nach Anspruch 1, **dadurch gekennzeichnet, dass** die Anordnung für jedes längliche Verbindungselement (17) ein Befestigungselement umfasst, um die Verankerung des Verbindungselements (17) in der inneren Wand sicherzustellen.

3. Anordnung nach Anspruch 2, **dadurch gekennzeichnet, dass** das Befestigungselement eine Verankerungsschraube ist, die das längliche Verbindungselement (17) axial verlängert und an dessen distalem Ende platziert ist.

4. Anordnung nach einem der Ansprüche 1 bis 3, **dadurch gekennzeichnet, dass** jedes längliche Verbindungselement (17) aus der Gruppe gewählt ist, umfassend ein längliches Kabel, einen Draht, eine Schnur, eine Stange und Kombinationen dieser Elemente.

5. Anordnung nach einem der Ansprüche 1 bis 4, **dadurch gekennzeichnet, dass** die Vorrichtung ferner einen Ring oder einen Flansch umfasst, der das distale Ende der Herzpumpe (12) umgibt, wobei wenigstens einige der länglichen Verbindungselemente mit einem ihrer Enden fest mit diesem Ring bzw. diesem Flansch verbunden sind.

6. Anordnung nach Anspruch 5, **dadurch gekennzeichnet, dass** die länglichen Verbindungselemente fest mit dem Ring oder dem Flansch verbunden sind, die aus dem Ring oder dem Flansch und den Verbindungselementen gebildete Anordnung aus einem Formgedächtnismaterial besteht, so dass diese Anordnung beim Einführen in die Kammer von einer nicht entfalteten Konfiguration in eine entfaltete Konfiguration übergeht, in der die freien Enden der Verbindungselemente an den inneren Wänden der Ventrikelkammer anliegen.

7. Anordnung nach einem der Ansprüche 1 bis 5, **dadurch gekennzeichnet, dass** jedes längliche Verbindungselement (17) ein längliches Kabel oder eine Stange ist, die Herzpumpe (12) Kabelkanäle (16) aufweist, die dazu ausgebildet sind, jeweils das Einführen eines entsprechenden länglichen Kabels oder einer Stange von außerhalb des Herzens in das Innere der Herzkammer zu ermöglichen, wenn die Pumpe an der Verankerungsvorrichtung (11) befestigt ist.

8. Anordnung nach Anspruch 7, **dadurch gekennzeichnet, dass** der Körper jedes länglichen Kabels oder jeder Stange dazu ausgebildet ist, eine Torsionssteifigkeit aufzuweisen, die die Übertragung einer von einem proximalen Ende dieses Kabels oder dieser Stange ausgeübten Drehbewegung über seine gesamte Länge gewährleistet.

9. Anordnung nach Anspruch 7 oder 8, **dadurch gekennzeichnet, dass** sich die Durchgangskanäle (16) des Kabels vom proximalen Ende der Herzpumpe (12) bis zu einem Teil der Pumpe erstrecken, der dazu bestimmt ist, im Inneren der Kammer platziert zu werden, wenn die Pumpe an der Verankerungsvorrichtung (11) befestigt ist, fest mit der Öffnung verbunden.

10. Anordnung nach einem der Ansprüche 7 bis 9, **dadurch gekennzeichnet, dass** wenigstens ein Teil wenigstens eines der Durchgangskanäle (16) in der Dicke der den Pumpenkörper begrenzenden Seitenwand platziert ist und/oder durch ein hohles, längliches Element begrenzt wird, das aus dem Pumpenkörper hervorsteht.

11. Anordnung nach einem der Ansprüche 7 bis 10, **dadurch gekennzeichnet, dass** jeder Durchgangskanal ein Rückschlagventil aufweist, um seine Dichtheit zu gewährleisten.

12. Anordnung nach einem der Ansprüche 1 bis 11, **dadurch gekennzeichnet, dass** die Pumpe eine Antriebsherzpumpe (12) ist.

## Claims

1. A ventricular assistance assembly for assisting a heart, said assembly comprising:
- a device (11) for anchoring a cardiac pump (12), intended to be assembled to an opening of a ventricular wall (13) of said heart, said anchoring device (11) delimiting an internal passage,
- a cardiac pump (12) intended to be attached on said anchoring device (11), said cardiac pump (12) being configured for an intraventricular insertion in said heart, said cardiac pump (12) having a distal end,
- said cardiac pump (12) extending through said internal passage to the inside of the heart when it is attached to said anchoring device (11) implanted in said opening, such that its distal end is placed in a ventricular chamber, **characterised in that** the assembly comprises:
- a device for stabilising said cardiac pump (12) comprising at least two flexible, biocompatible, elongated connection members, each elongated connection member (17) being intended to connect a portion of said cardiac pump (12) located in said ventricular chamber to an internal wall of said ventricular chamber.

2. The assembly according to claim 1, **characterised in that** for each elongated connection member (17), the assembly comprises an attachment element to ensure the anchoring of said connection member (17) in said internal wall.

3. The assembly according to claim 2, **characterised in that** said attachment element is an anchoring screw axially extending the elongated connection member (17) and placed at its distal end.

4. The assembly according to any one of claims 1 to 3, **characterised in that** each elongated connection member (17) is chosen from the group comprising an elongated cable, a wire, a cord, a rod, and combinations of these elements.

5. The assembly according to any one of claims 1 to 4, **characterised in that** said assembly also comprises a ring, or a flange, intended to surround the distal end of the cardiac pump (12), at least some of the elongated connection members being secured by one of their ends to this ring, or respectively this flange.

6. The assembly according to claim 5, **characterised in that** said elongated connection members being secured to said ring or said flange, the assembly formed of said ring or said flange and said connection members is made from a shape memory material, such that when being introduced into the chamber, this assembly switches from a non-deployed configuration to a deployed configuration wherein the free ends of the connection members bear against the internal walls of said ventricular chamber.

7. The assembly according to any one of claims 1 to 5, **characterised in that** each elongated connection member (17) being either an elongated cable or a rod, said cardiac pump (12) includes cable passage channels (16) configured to each allow the insertion of a corresponding elongated cable, or a rod, from the outside of the heart to the inside of said ventricular chamber when said pump is attached to the anchoring device (11).

8. The assembly according to claim 7, **characterised in that** the body of each elongated cable or rod is intended to have torsional rigidity ensuring the transmission along its entire length of a rotational movement imparted from a proximal end of this cable or this rod.

9. The assembly according to claim 7 or 8, **characterised in that** the cable passage channels (16) extend from the proximal end of the cardiac pump (12) to a portion of said pump intended to be placed inside said chamber when said pump is attached to the anchoring device (11) secured to said opening.

10. The assembly according to any one of claims 7 to 9, **characterised in that** at least a portion of at least one of said passage channels (16) is located in the thickness of the side wall delimiting the pump body and/or is delimited by a hollow elongated element protruding from the pump body.

11. The assembly according to any one of claims 7 to 10, **characterised in that** each passage channel includes a non-return valve to ensure its tightness.

12. The assembly according to any one of claims 1 to 11, **characterised in that** said pump is a propulsive cardiac pump (12).
